# DEMANDE DE BREVET EUROPEEN

(11) **EP 0 707 839 A1**
(43) Date de publication de la demande: **24.04.1996**
(21) Numéro de dépôt: 95402203.4
(22) Date de dépôt: 02.10.1995
(51) Int. Cl.: A61F 2/38

(54) **Prothèse fémorale d'articulation du genou**

(30) Priorité: 17.10.1994 FR 9412347
(71) Demandeur: FRANCE-BLOC, F-95270 Viarmes (FR)
(72) Inventeur: Charpenet, Rémy, F-88150 Thaon-les-Vosges (FR); Grosdidier, Gilles, F-54000 Nancy (FR); Coudane, Henry, F-54000 Nancy (FR); Junger, Jean-Claude, F-57570 Cattenom (FR)
(74) Mandataire: Santarelli, Marc

(57) **Abrégé**

Prothèse fémorale d'articulation du genou comprenant un corps d'appui muni d'une partie trochléenne (1) antérieure, d'une partie médiane (2) et de deux parties condyliennes (3) postérieures, ledit corps d'appui se présentant sous la forme d'une bande courbée comportant un évidement central (4) constituant ainsi deux parties (5a,5b) analogues à des condyles, chacune des deux bandes comportant un plot d'ancrage à proximité de la partie médiane dudit corps d'appui, dans laquelle les bandes (5a,5b) condyliennes postérieures comportent une surépaisseur (6a,6b) vers l'intérieur de la prothèse, augmentant ainsi l'angle de flexion d'un fémur comportant une telle prothèse avant que ne se produise le blocage de la flexion par effet de came sur un élément prothétique tibial.

## Description

La présente demande concerne une prothèse tri-compartimentale d'articulation du genou, et en particulier sa pièce fémorale de première intention.

De nombreuses prothèses d'articulation du genou sont déjà connues. Celles-ci comprennent classiquement un élément fémoral et un élément tibial, tous deux de préférence métalliques, que l'on fixe sur les os correspondants.

Ces prothèses comprennent de plus un élément généralement réalisé en matière plastique comme un polyéthylène, qui recrée la surface articulaire du tibia et que l'on intercale entre les éléments fémoral et tibial.

L'ensemble des trois pièces précitées constitue ce que l'on appelle une prothèse tri-compartimentale.

De telles prothèses sont décrites par exemple dans EP-A-183 760, FR-A-2 290 883, ou US-A-4 586 933.

Les prothèses modernes reconstruisent les surfaces articulaires. Parmi les prothèses les plus récentes, on peut citer celles commercialisées par la Société HOWMEDICA sous la référence "KINEMATIC KNEE SYSTEM". Cette prothèse offre trois options, avec conservation des ligaments croisés, avec conservation du ligament croisé postérieur, ou sans conservation des ligaments croisés. Toutes ces options permettent au chirurgien d'effectuer le choix biomécanique le mieux adapté à la situation médicale.

La prothèse PCA possède pour sa part des condyles asymétriques comprenant un plateau tibial échancré à la partie postérieure permettant la conservation du ligament croisé postérieur. Le bouton rotulien est asymétrique et comporte, au niveau de la zone de contact osseux, un revêtement poreux permettant son implantation sans utilisation de ciment. Tout comme le fémur naturel, le composant fémoral présente des condyles divergents et des rayons de courbure différents.

On peut également citer la prothèse RMC de la série TOTAL KNEE SYSTEM, qui est constituée d'une pièce fémorale asymétrique comprenant des pièces tibiales permettant de conserver ou non les ligaments croisés. La rotule a une forme asymétrique, proche de la forme anatomique.

EP-A-0 600 806 décrit une prothèse tri-compartimentale du genou comportant un implant fémoral et un implant tibial, l'implant fémoral étant du type de ceux présentant une échancrure délimitant deux patins condyliens d'appui réunis par une trochlée, dans laquelle l'implant fémoral a une forme anatomique résultant de la combinaison d'un certain nombre de caractéristiques. La longueur de la courbure postérieure est plus grande sur les patins condyliens, de sorte que leur épaisseur au niveau de cette partie postérieure, donc vers l'extérieur de la prothèse, est plus importante que celle connue à ce jour sur les prothèses existantes ; cette caractéristique est présentée comme améliorant la flexion.

EP-A-0 567 705 concerne une prothèse totale postéro-stabilisée du genou comprenant un élément fémoral, deux condyles, et une butée de postéro-stabilisation orientée vers l'avant et occupant en partie l'espace intercondylien.

L'évolution des prothèses montre que les diverses sociétés impliquées dans ce type de recherche ont pour objectif de fournir des pièces prothétiques se rapprochant le plus possible de l'anatomie humaine, afin de mieux respecter la cinématique ligamentaire et obtenir ainsi les meilleures performances.

Les problèmes majeurs rencontrés lors de la mise en point de prothèses fémorales d'articulation sont la conservation maximum d'os dur, dans lequel l'ancrage de la prothèse s'effectue dans de bonnes conditions, et la limitation de l'angle de flexion du genou.

En effet la fixation de la partie fémorale de la prothèse dans le fémur s'accompagne toujours d'une résection osseuse importante, et de ce fait on arrive souvent à l'os spongieux. La fixation d'une telle prothèse dans un élément spongieux est, bien évidemment, moins efficace qu'une installation dans de l'os dur. De plus, il est souhaitable que l'installation d'une prothèse permette aux malades ainsi appareillés d'avoir une amplitude de flexion du genou la plus proche possible de l'amplitude physiologique.

C'est pourquoi la présente demande a pour objet une prothèse fémorale monobloc d'articulation du genou comprenant un corps d'appui muni d'une partie trochléenne antérieure, d'une partie médiane et de deux parties condyliennes postérieures, ledit corps d'appui se présentant sous la forme d'une bande courbée comportant un évidement central constituant ainsi deux bandes analogues à des condyles, chacune des deux bandes comportant un plot d'ancrage à proximité de la partie médiane dudit corps d'appui, caractérisée en ce que les parties condyliennes postérieures comportent une surépaisseur vers l'intérieur de la prothèse augmentant ainsi l'angle de flexion d'un fémur comportant une telle prothèse avant que ne se produise le blocage de la flexion par effet de came sur un élément prothétique tibial, ladite surépaisseur correspondant à un prolongement (A) de la courbure postérieure des condyles de 3 mm à 10 mm vers l'intérieur de la prothèse.

En effet, après de longues recherches la demanderesse a découvert qu'en conservant une conformation anatomique, mais en conférant aux bandes condyliennes une surépaisseur postérieure en combinaison en conséquence avec une coupe postérieure osseuse plus importante que celle nécessitée dans l'art antérieur, on obtenait un effet de came plus tardif que celui obtenu avec les prothèses de l'art antérieur. On obtient ainsi, notamment pour une flexion supérieure à 60°, une grande surface de contact avec le plateau tibial.

Par prothèse fémorale "monobloc", l'on entend qu'il s'agit d'une prothèse de première intention, et non une prothèse de révision pouvant être ajustée à l'aide de cales de dimensions adaptées au jeu à compenser.

Comme on l'a vu ci-dessus, la surépaisseur précitée correspond à un prolongement (A) de la courbure postérieure des condyles de 3 mm à 10 mm vers l'intérieur de la prothèse. On verra ci-après sur les figures la signification et l'illustration de cet allongement (A). Par exemple au niveau de sa courbure maximale, l'épaisseur maximum de la partie condylienne de la prothèse selon l'invention peut aller de 11,5 à 16,5 mm selon la taille du fémur ; par exemple pour un fémur moyen, cette épaisseur sera d'environ 14,5 mm. De plus la prothèse fémorale selon l'invention a une partie médiane d'une épaisseur inhabituellement réduite. L'épaisseur e de la partie médiane, peut avoir par exemple de 5 mm à 8 mm, et de préférence cette épaisseur "e" ne dépasse pas 6 mm.

Afin d'obtenir cette épaisseur dans la partie médiane particulièrement réduite en tous points, la prothèse selon l'invention est caractérisée en ce que la partie médiane comprend, dans le sens de la largeur, et vers l'intérieur de ladite prothèse quatre surfaces en contact avec l'os, deux de celles-ci, plus externes et sensiblement coplanaires sont aplaties et forment un plan sensiblement perpendiculaire à l'axe fémoral, et les deux autres, internes et courbes sont dirigées vers l'intérieur du fémur à savoir vers la partie médiane de l'espace intercondylien, les deux surfaces externes étant situées de 5 à 10 mm en dessous du niveau supérieur de la partie médiane de la prothèse.

Des prothèses préférées parmi les prothèses ci-dessus sont celles caractérisées en ce qu'en outre la partie intermédiaire entre la partie médiane et l'extrémité trochléenne comprend également des surfaces aplaties, analogues aux deux surfaces aplaties externes mais placées en position oblique par rapport à celles-ci.

Dans un tel cas, des surfaces courbes dans le prolongement à la fois desdites surfaces aplaties analogues, et deux surfaces courbes ci-dessus sont également prévues, et sont alors en continuité l'une avec l'autre.

Des prothèses tout particulièrement préférées sont des prothèses caractérisées en ce que de surplus, les surfaces aplaties définies ci-dessus forment un angle d'environ 45° par rapport à celles définies précédemment et l'extrémité condylienne postérieure comprend également des surfaces aplaties, analogues aux deux surfaces aplaties externes mais placées en position oblique par rapport à celles-ci.

Comme on le verra illustré sur les figures, les surfaces aplaties médianes externes forment un angle d'environ 40°, par exemple 35 à 50° par rapport aux parties analogues aplaties situées au niveau des parties intermédiaires entre la partie médiane et d'une part l'extrémité trochléenne et d'autre part, l'extrémité condylienne postérieure.

Dans une variante, toutes les surfaces courbes définies ci-dessus peuvent, en totalité ou en partie, être planes.

La présente demande a également pour objet une prothèse tri-compartimentale de genou, caractérisée en ce qu'elle comporte une prothèse fémorale telle que définie ci-dessus.

L'invention sera mieux comprise si l'on se réfère aux dessins annexés sur lesquels :

La figure 1 représente une vue en perspective d'une prothèse fémorale droite d'articulation du genou selon la présente invention.

La figure 2 représente une prothèse fémorale droite selon l'invention vue de face du coté de la trochlée.

La figure 3 est une vue en coupe selon BB' de la figure 1.

La figure 4 représente une prothèse fémorale droite selon l'invention vue de profil.

La figure 5 est une vue en perspective de surfaces internes (donc en contact avec l'os fémoral) d'une prothèse selon l'invention.

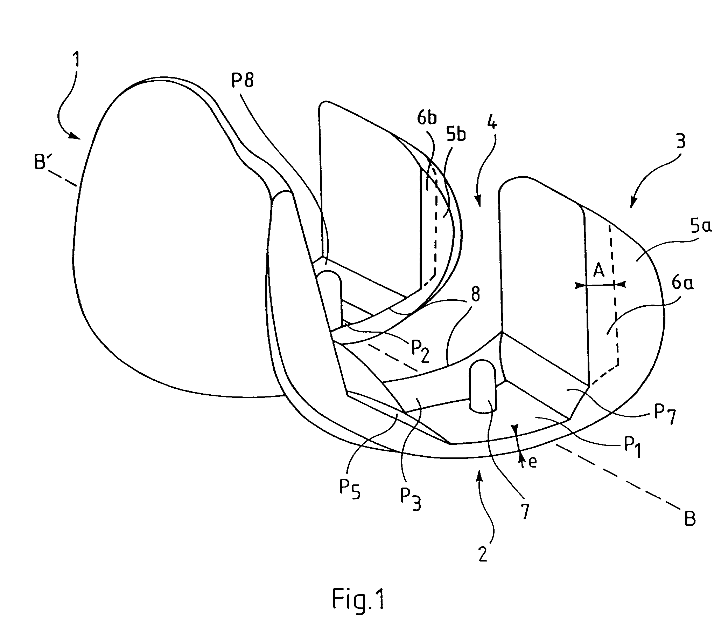

Sur la figure 1 on distingue des éléments classiques d'une prothèse fémorale d'articulation du genou. Celle-ci comprend une partie trochléenne antérieure 1, une partie médiane 2, et deux parties condyliennes postérieures 3. Le corps d'appui se présente sous la forme générale d'une bande courbée, à courbure asymétrique, de rayon plus important du côté trochléen. Cette bande courbée comporte un évidement central 4 constituant deux bandes analogues à des condyles. Chacune de ces bandes comporte un plot d'ancrage 7 à proximité de la partie médiane du corps d'appui.

Ces éléments conventionnels comportent un certain nombre de particularités qui sont exposées ci-après. Les bandes condyliennes (5a, 5b) selon l'invention comportent sur la partie postérieure condylienne une surépaisseur (6a, 6b) vers l'intérieur de la prothèse. Cette surépaisseur représentée par A sur la figure a pour effet d'augmenter l'angle de flexion d'un fémur comportant une telle prothèse avant que le blocage de la flexion n'intervienne par effet de came sur la partie tibiale d'un élément prothétique. Les prothèses selon l'invention ont donc la surface de leurs condyles prolongée par rapport à des prothèses conventionnelles. Ce prolongement A peut aller de 3 mm à 10 mm selon la taille. Il coopère avec une coupe osseuse correspondante plus importante à ce niveau que dans l'art antérieur.

En effet, pour une prothèse classique, la coupe postérieure est de l'ordre de 8 mm. Pour la prothèse objet de la présente invention, cette coupe est plus importante mais ne fragilise pas sensiblement le support osseux à ce niveau. L'épargne osseuse présente ici un intérêt moins important que le gain en flexion qui apporte un confort important lors de la marche.

La prothèse selon l'invention répond à un second objectif : elle permet d'avoir un ancrage qui se situe dans la partie solide de l'os, évitant au maximum une fixation dans de l'os spongieux.

Ainsi la prothèse selon l'invention est caractérisée en ce que sa partie médiane 2 comprend dans le sens de la largeur et à l'intérieur de ladite prothèse, c'est à dire du côté opposé aux surfaces de glissement, deux surfaces aplaties : P1 et P2, et deux surfaces arrondies : P3 et P4, P4 n'étant pas visible sur cette figure 1 mais correspondant à la partie P3. Les deux premières, P1 et P2, externes par rapport à la prothèse, sont sensiblement coplanaires et forment ainsi un plan sensiblement perpendiculaire à l'axe fémoral. Ces deux surfaces aplaties externes ont la particularité d'être situées de (2 mm à 10 mm) en dessous du niveau supérieur 8 de la partie médiane de la prothèse. Les deux surfaces arrondies, P3 et P4, internes par rapport à la prothèse sont donc dirigées vers l'intérieur du fémur, à savoir vers la partie médiane 4 de l'espace inter-condylien.

On distingue également sur cette figure une autre partie aplatie, P5, la partie analogue P6 étant cachée par la trochlée. Ces parties P5 et P6 sont sensiblement dans la continuité, respectivement, des surfaces aplaties P1 et P2.

Ces parties P5 et P6 sont placées en position oblique par rapport aux deux surfaces aplaties externes P1 et P2. Par "placées en position oblique", on entend que les plans font un certain angle ; par exemple, sur la figure, on observe que les plans P1 et P5 forment un angle d'environ 45°.

Si désiré, on trouve de ce côté également d'autres surfaces courbes P9 et P10, correspondant aux parties P3 et P4.

Cette zone est une zone continue, a savoir que les surfaces gauche et droite (ou interne et externe) de la prothèse ne sont pas séparées à ce niveau par l'évidement central 4.

Sur la figure 1, on distingue également du côté condylien d'autres surfaces planes, dans la continuité de P5 et de P1, ainsi que de P6 et de P2. Les surfaces analogues aux parties P1 et P2 sont, sur la figure, les parties P7 et P8. De préférence, la surface de ces parties P7 et P8 est sensiblement plus réduite que celles des parties P1 et P2. En particulier les surfaces des zones P1 et P7 pourraient être par exemple dans un rapport de 5 à 1. Les caractéristiques générales de ces bandes P7 et P8 sont analogues à celles des bandes P5 et P6, à savoir qu'elles sont en continuité avec les bandes P1 et P2 et sont placées en position oblique par rapport à celles-ci. Elles sont toutefois plus effilées vers l'intérieur de la prothèse en raison de l'absence de surfaces analogues aux surfaces P3 et P4.

Comme on peut l'observer sur la figure, l'épaisseur e, en tout point de la partie médiane, est généralement inférieure à 8 mm, et en général égale à 6 mm environ.

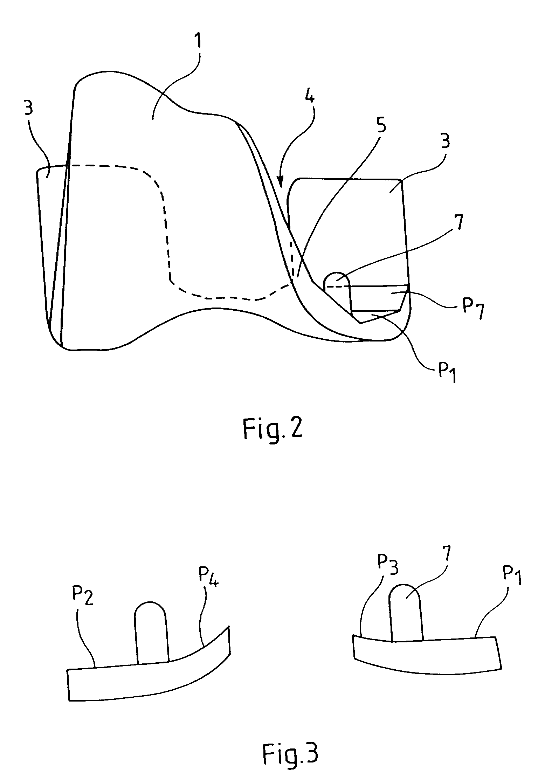

Sur la figure 2, on observe la trochlée 1 en premier plan et, en arrière plan, les condyles 3. On peut observer sur cette figure l'importante économie osseuse réalisée au niveau de l'ancrage de la prothèse grâce à la conception de la prothèse selon la présente invention.

En effet, dans une prothèse conventionnelle, la résection osseuse se fait au-dessus du point désigné par S sur la figure. Dans la présente invention, la totalité du plot d'ancrage 7 se trouve incorporée dans de l'os dur, et non dans de l'os spongieux. En effet, comme on le voit sur la figure, la partie extrême du plot est à peine au delà du plan délimité par le point désigné par S.

Sur la figure 3, on distingue les deux plots 7, placés sur les surfaces planes P1 et P2. On distingue également les surfaces courbes P3 et P4.

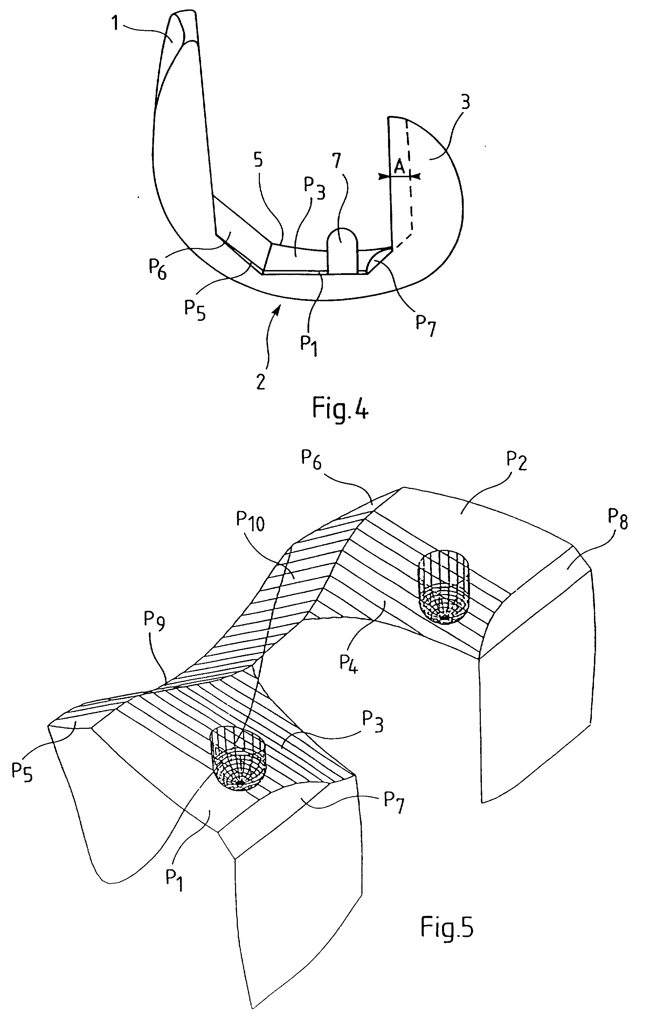

La figure 4 représente une prothèse selon l'invention vue de profil, du côté intérieur. On distingue les mêmes éléments que précédemment et en particulier la surépaisseur A d'un condyle 3 ainsi que le degré important de l'épargne d'os dur, constituée par un volume délimité par un plan horizontal passant par le point désigné S, vers le haut et délimité vers le bas par les surfaces P1, P3, P5, P6 et P7.

La figure 5 est une vue en perspective informatisée des surfaces internes de la prothèse selon l'invention (ou de la résection fémorale correspondante). Les surfaces courbes sont indiquées par des hachures.

En extension la force appliquée au fémur est transmise verticalement. Les surfaces transmettant l'effort sont la surface distale et le chanfrein antérieur.

La configuration au niveau distal de la présente invention permet de conserver le maximum d'os dur. Cet os dur offre une surface d'appui très résistante pour la prothèse fémorale et évite ainsi l'enfoncement de la prothèse dans l'os spongieux.

En flexion à 90 degrés, l'effort transmis est moins important. Cette fois le fémur n'est plus soumis à un effort de compression mais à un effort de cisaillement. La surface transmettant l'effort est la surface de la coupe postérieure des condyles.

La densité de l'os de la coupe postérieure des condyles étant sensiblement constante entre la coupe classique et la coupe nécessitée par la prothèse selon l'invention, on n'a pas de fragilisation du support osseux.

## Revendications

1. Prothèse fémorale monobloc d'articulation du genou comprenant un corps d'appui muni d'une partie trochléenne (1) antérieure, d'une partie médiane (2) et de deux parties condyliennes (3) postérieures, ledit corps d'appui se présentant sous la forme d'une bande courbée comportant un évidement central (4) constituant ainsi deux parties (5a, 5b) analogues à des condyles, chacune des deux bandes comportant un plot d'ancrage à proximité de la partie médiane dudit corps d'appui, caractérisée en ce que, comme représenté sur les figures, les bandes (5a, 5b) condyliennes postérieures comportent une surépaisseur (6a, 6b) vers l'intérieur de la prothèse, augmentant ainsi l'angle de flexion d'un fémur comportant une telle prothèse avant que ne se produise le blocage de la flexion par effet de came sur un élément prothétique tibial, ladite surépaisseur correspondant à un prolongement (A) de la courbure postérieure des condyles de 3 mm à 10 mm vers l'intérieur de la prothèse.

2. Prothèse selon la revendication 1, caractérisée en ce que la partie médiane (2) a une épaisseur ne dépassant pas 6 mm environ.

3. Prothèse selon la revendication 2, caractérisée en ce que la partie médiane (2) comprend, dans le sens de la largeur, et à l'intérieur de ladite prothèse quatre surfaces en contact avec l'os, deux de celles-ci (P1, P2), aplaties, plus externes et sensiblement coplanaires, formant un plan sensiblement perpendiculaire à l'axe fémoral, et les deux autres (P3, P4), internes et courbes ou planes sont dirigées vers l'intérieur du fémur à savoir vers la partie médiane de l'espace intercondylien (4), les deux surfaces externes étant situées de 5 à 10 mm en dessous du niveau supérieur (8) de la partie médiane de la prothèse.

4. Prothèse selon la revendication 3, caractérisée en ce qu'en outre, la partie intermédiaire entre la partie médiane (2) et l'extrémité trochléenne comprend également des surfaces aplaties (P5, P6), analogues aux deux surfaces aplaties externes (P1, P2) mais placées en position oblique par rapport à celles-ci.

5. Prothèse selon la revendication 3 ou 4, caractérisée en ce que les surfaces aplaties définies à la revendication 4 forment un angle d'environ 45° par rapport à celles définies à la revendication 5 et l'extrémité condylienne postérieure comprend également des surfaces aplaties (P7, P8), analogues aux deux surfaces aplaties externes (P1, P2) mais placées en position oblique par rapport à celles-ci.

6. Prothèse selon la revendication 3 ou 4, caractérisée en ce que les surfaces aplaties (P1, P2) définies à la revendication 4 forment un angle d'environ 40° par rapport à celles définies à la revendication 4 ou 5.

7. Prothèse tri-compartimentale de genou, caractérisée en ce qu'elle comporte une prothèse fémorale définie à la revendication 1.

8. Prothèse tri-compartimentale de genou, caractérisée en ce qu'elle comporte une prothèse fémorale définie à la revendication 2 ou 3.

9. Prothèse tri-compartimentale de genou, caractérisée en ce qu'elle comporte une prothèse fémorale définie à la revendication 4 ou 5.
